# FASCICULE DE BREVET EUROPEEN

(11) **EP 3 374 774 B1**
(45) Date de publication et mention de la délivrance du brevet: **04.05.2022**
(21) Numéro de dépôt: 16809975.2
(22) Date de dépôt: 09.11.2016
(51) Int. Cl.: G01N 35/00, G01N 35/10, G01N 1/00, G01N 35/04

(54) **DISPOSITIF D'AGITATION ET DE PRÉLÈVEMENT D'ÉCHANTILLONS DE LIQUIDES BIOLOGIQUES ADAPTÉ POUR TRIER**
VORRICHTUNG ZUM SCHÜTTELN UND ENTNEHMEN BIOLOGISCHER FLÜSSIGKEITSPROBEN MIT EIGNUNG ZUM SORTIEREN
DEVICE FOR AGITATING AND COLLECTING BIOLOGICAL LIQUID SAMPLES SUITABLE FOR SORTING

(30) Priorité: 13.11.2015 FR 1560890
(43) Date de publication de la demande: 19.09.2018
(73) Titulaire: Horiba ABX SAS / Horiba Ltd, 34184 Montpellier cedex 4 (FR)
(72) Inventeur: BEAUDUCEL, Florent, 34070 Montpellier (FR); BENEZETH, Philippe, 30132 Caissargues (FR)
(74) Mandataire: Cabinet Netter
(86) Numéro de dépôt international: PCT/FR2016/052912
(87) Numéro de publication internationale: WO 2017/081411

(56) Documents cités:
- EP-A1- 0 726 453
- EP-A1- 1 655 071
- EP-A2- 2 693 220
- WO-A1-93/25885
- WO-A1-03/100382
- FR-A1- 2 888 328

## Description

L'invention concerne le domaine du prélèvement d'échantillons de liquides biologiques en particulier un fluide corporel, par exemple du sang.

Au cours des dernières décennies, les appareils d'analyse des produits sanguins ont connu une automatisation croissante. Afin de satisfaire les demandes d'analyses sanguines, les besoins en capacité de prélèvement et de multi-analyse ont explosé. Cela se traduit par le fait que les appareils modernes sont évalués en fonction de leur cadence d'analyse (nombre de tubes par heure), et de leur capacité à réaliser plusieurs types de tests sur une seule machine.

Les développements du domaine ont d'abord favorisé la cadence de traitement, puis l'ajout de différents types de tests. Certaines tâches de la séquence à réaliser par le dispositif viennent contraindre la capacité à augmenter la cadence par l'ajout de ressources matérielles.

Ainsi, il est nécessaire d'agiter les tubes un certain temps afin que le produit sanguin prélevé soit homogène au moment du prélèvement. À l'inverse, un échantillon agité ne doit pas rester trop longtemps au repos avant le prélèvement, sous peine de perdre les bénéfices de l'agitation. De plus, l'agitation ne peut pas être accélérée au-delà d'une certaine vitesse, sinon l'échantillon risque d'être détérioré et/ou le mélange obtenu sera insatisfaisant.

Afin d'augmenter la cadence en tenant compte de ces contraintes, il a été prévu des agitateurs à deux tubes, puis des supports de tubes, également appelés portoirs, afin d'agiter plusieurs tubes en même temps. Les portoirs ont été conçus avec divers agencements, jusqu'à atteindre les limites liées à l'agitation et à l'encombrement.

Ensuite, les agitateurs ont été modifiés afin de pouvoir recevoir deux portoirs simultanémenl, aboutissant ainsi à des dispositifs tels que celui décrit dans la demande de brevet EP 0 726 453 A1, qui correspond au préambule de la revendication 1.

Ainsi, le maintien de la cadence a été amélioré lors de la transition entre deux portoirs successifs, tout en permettant de maintenir une agitation satisfaisante. Progressivement, les dispositifs ont été automatisés jusqu'aux limites du traitement séquentiel des portoirs. Cependant, ces améliorations ont maintenant atteint leurs limites en termes de cadence pure, mais également en termes de gestion de cas exceptionnels.

Par cas exceptionnel, on entend par exemple lorsqu'un portoir ne contient qu'un tube, ou lorsqu'un tube doit être examiné en urgence, sans tenir compte des portoirs déjà traités, ou encore lorsqu'un tube ayant déjà fait l'objet d'un prélèvement et d'une analyse doit être analysé à nouveau car une suspicion existe sur le résultat d'une analyse (par exemple, mesure à la marge d'un seuil de détection, etc.).

La situation du « tube unique » se retrouve également lorsqu'un portoir contient des tubes demandant des analyses non gérées par le dispositif, et qu'un seul tube sur un portoir peut être effectivement analysé par le dispositif.

Cette augmentation des cadences s'accompagne de l'intégration croissante de dispositifs au sein d'une même installation. La gestion de la répartition des tubes entre les divers dispositifs, ainsi que leur archivage une fois les tests souhaités réalisés se pose de manière pressante.

Pour résoudre ce problème, des appareils spécialisés, appelés « trieurs » ont été développés. Ces appareils ont pour unique fonction le tri de portoirs de tubes afin de regrouper ensemble les tubes destinés à des emplacements similaires, comme l'archivage.

Ces dispositif sont très encombrants, posent des problèmes importants de dimensionnement, représentent un surcoût conséquent. En plus de ces défauts, dès que l'installation change de taille, les trieurs deviennent sur ou sous dimensionnés, constituant ainsi synonyme d'inadéquation ou goulot d'étranglement.

À cet effet, telle que définie dans la revendication 1, propose un dispositif d'agitation et de prélèvement d'échantillons de liquides biologiques comprenant un agitateur de portoirs de tubes, un organe d'échantillonnage propre à réaliser un prélèvement d'échantillon de liquides biologiques dans un tube, et un passeur capable de saisir un tube sur un portoir reçu dans l'agitateur et de le déplacer jusqu'à l'organe d'échantillonnage.

L'agitateur est capable d'agiter au moins trois portoirs simultanément, et le dispositif comporte en outre un ordonnanceur capable de déterminer des données de destination pour un tube et des données de destination pour le portoir qui reçoit ce tube, et de déterminer pour chaque tube un emplacement final en fonction des données de destination du tube et des données de destination des portoirs reçus dans le dispositif, lequel emplacement final désigne un portoir reçu sur l'agitateur et une position sur ce portoir et peut être différent de l'emplacement du tube lorsque le portoir qui le recevait a été introduit dans le dispositif, et agencé pour commander le passeur pour saisir un tube, le présenter à l'organe d'échantillonnage et le replacer après prélèvement audit emplacement final.

Ce dispositif est particulièrement avantageux car il vient totalement changer la manière dont est envisagée la gestion des tubes. En effet, dans l'art antérieur, les tubes étaient toujours analysés de manière séquentielle selon leur emplacement dans les portoirs, de même que les portoirs étaient toujours traités de manière séquentielle. Les dispositifs étaient vus comme des machines mécaniques automatisées qui devaient traiter le plus rapidement possibles les portoirs que l'utilisateur introduisait.

L'invention vient changer radicalement la manière dont fonctionnent les dispositifs de prélèvement, en permettant un traitement non plus strictement séquentiel, mais indexé pour maintenir une cadence pleine quelle que soit la situation. En effet, l'ordonnanceur permet de traiter les tubes sur les portoirs selon un ordre intelligent dépendant de l'état général de traitement du dispositif, mais également en fonction des opérations et analyses à réaliser sur les tubes, ou encore en fonction d'autres paramètres.

Il n'est plus question de machines mécaniques automatisées traitant les tubes les uns après les autres le plus rapidement possible, mais de machine intelligente s'adaptant aux conditions opérationnelles afin de maintenir une cadence maximale, quelles que soient ces conditions.

Selon diverses variantes, le dispositif pourra présenter une ou plusieurs des caractéristiques suivantes :
- l'ordonnanceur est agencé, pour un tube donné, pour choisir l'emplacement final parmi les emplacements libres sur un portoir qui présente les mêmes données de destination que le tube donné,
- l'ordonnanceur est agencé pour déterminer comme emplacement final sur un portoir donné le premier emplacement vide sur le portoir donné,
- l'ordonnanceur est agencé pour mettre à zéro les données de destination associées à un portoir donné lors de son introduction dans le dispositif,
- l'ordonnanceur est agencé, lorsqu'aucun portoir ne présente les mêmes données de destination qu'un tube saisi par le passeur, pour affecter les données de destination associées à ce tube à un portoir auquel aucune donnée de destination n'est associée,
- l'ordonnanceur est agencé pour commander le passeur pour décaler verticalement un tube reçu dans un portoir en fonction des données de destination associées à ce tube,
- l'ordonnanceur est agencé pour commander le passeur pour faire sortir un portoir du dispositif, et pour le disposer en une position de sortie variant en fonction des données de destination associées au portoir,
- l'ordonnanceur est agencé pour faire sortir un portoir du dispositif présentant des emplacements vides, en réponse à une commande utilisateur ou en réponse au dépassement d'une durée choisie,
- l'ordonnanceur est agencé, après avoir fait sortir un portoir présentant des emplacements vides, pour faire rentrer dans le dispositif un portoir présentant un nombre d'emplacements vides au moins égal, et
- le dispositif comprend en outre une zone pour connexion à un convoyeur de portoirs.

L'invention concerne également un procédé d'agitation et de prélèvement de tubes, comprenant les opérations suivantes :
a. introduire au moins un portoir recevant au moins un tube contenant au moins un liquide biologique dans un dispositif d'agitation et de prélèvement d'échantillons de liquides biologiques,
b. déterminer des données de destination pour chaque tube dans le dispositif et des données de destination pour chaque portoir qui reçoit un de ces tubes,
c. déterminer pour chaque tube reçu dans le dispositif un emplacement final en fonction des données de destination du tube et des données de destination des portoirs reçus dans le dispositif, lequel emplacement final désigne un portoir reçu dans le dispositif et une position sur ce portoir, et peut être différent de l'emplacement du tube lorsque le portoir qui le recevait a été introduit dans le dispositif
d. réaliser le prélèvement d'échantillons de liquides biologiques dans chaque tube reçu dans le dispositif,
e. placer le tube à son emplacement final après l'opération d).

D'autres caractéristiques et avantages de l'invention apparaîtront mieux à la lecture de la description qui suit, tirée d'exemples donnés à titre illustratif et non limitatif, tirés des dessins sur lesquels :
- la figure 1 représente une vue en perspective d'un dispositif selon l'invention,
- la figure 2 représente une vue en perspective d'une partie des éléments du dispositif de la figure 1,
- la figure 3 représente une vue de côté de l'intérieur du dispositif de la figure 1,
- les figures 4 et 5 représentent respectivement une vue en perspective et une vue de côté d'un agitateur représenté sur les figures 2 et 3,
- les figures 6 et 7 représentent une vue en perspective d'un portoir de tubes des figure 4 et 5 respectivement sans et avec tube,
- la figure 8 représente une vue en perspective du passeur de la figure 2,
- la figure 9 représente un exemple d'une fonction mise en œuvre par l'ordonnanceur, et
- la figure 10 représente une vue schématique d'une installation comprenant plusieurs dispositifs dont l'un au moins est selon la figure 1.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

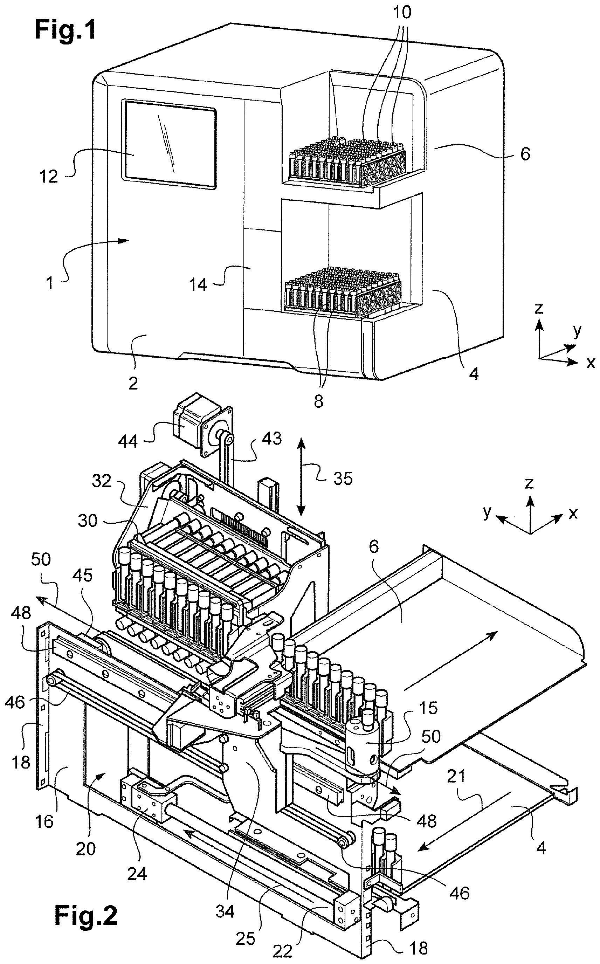

La figure 1 représente une vue en perspective d'un dispositif 1 selon l'invention. Un repère XYZ permet de repérer les diverses orientations sur les figures sur lesquels il est reproduit.

Le dispositif 1 comprend un boîtier 2 de forme générale parallélépipédique, qui présente deux évidements respectivement pour une tablette de chargement 4 et une tablette de déchargement 6. Les tablettes 4 et 6 permettent respectivement d'introduire et d'extraire des portoirs 8 de tubes 10 contenant les liquides biologiques comme des produits sanguins que le dispositif 1 doit échantillonner et analyser. Les portoirs 8 sont disposés parallèlement les uns aux autres sur les tablettes 4 et 6, dans le sens de leur longueur, c'est-à-dire selon l'axe Y.

Dans l'exemple représenté sur la figure 1, les portoirs sont introduits dans la machine de la droite vers la gauche sur la tablette de chargement 4, c'est-à-dire que c'est le portoir 8 le plus à gauche de la tablette de chargement 4 qui sera inséré le premier, et le plus à droite qui sera inséré en dernier. Pour la tablette de déchargement 6, la situation est inverse : c'est le portoir 8 le plus à droite qui a été éjecté en premier, tandis que le portoir 8 le plus à gauche est celui qui a été éjecté le plus récemment. Dans l'exemple décrit ici, du fait de l'agencement intérieur du dispositif 1, la tablette de chargement 4 est située dans la partie inférieure du dispositif 1, tandis que la tablette de déchargement 6 est située dans la partie supérieure. En variante, les fonctions des tablettes 4 et 6 pourraient être inversées, ou les tablettes pourraient avoir des rôles dépendant du mode de fonctionnement du dispositif 1, en servant au chargement ou au déchargement des portoirs 8 en fonction de la situation.

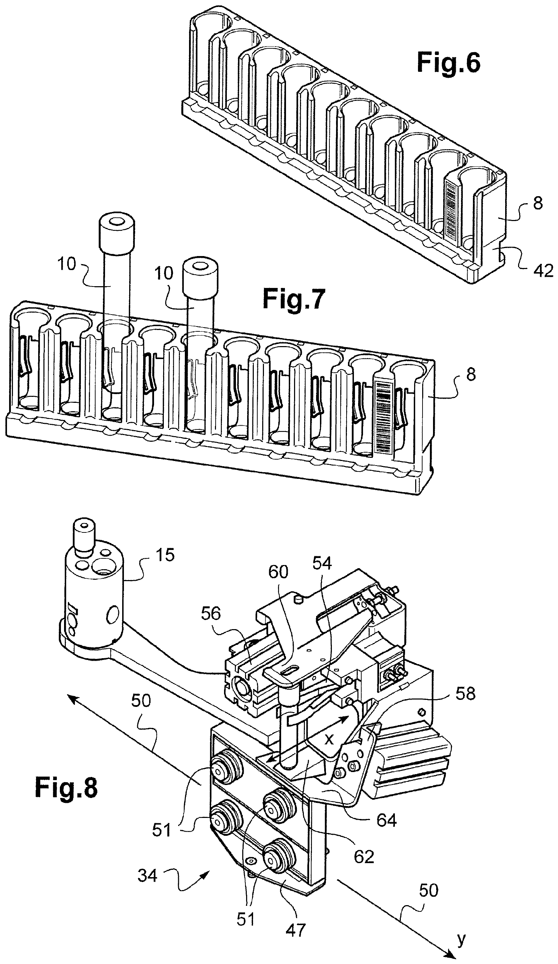

Le boîtier 2 présente également un écran 12 permettant de commander le dispositif 1 ainsi que de suivre l'état de fonctionnement de celui-ci. Enfin, le boîtier 2 présente une trappe 14 qui permet l'introduction d'un « tube urgent ». Un tube urgent est un tube unique qu'un utilisateur souhaite traiter en priorité par rapport aux autres. Comme on peut le voir sur les figures 2 et 8, ce tube est reçu dans un barillet 15 qui fait partie du dispositif 1. La trappe 14 peut aussi être utilisée pour introduire des tubes dont le traitement est incompatible avec l'utilisation d'un portoir, par exemple des tubes pédiatriques.

La figure 2 représente une vue en perspective d'une partie des éléments contenus à l'intérieur du boîtier 2 du dispositif 1. Comme on peut le voir sur cette figure, le dispositif 1 reçoit une paroi 16 qui s'exprime principalement selon la direction Y et est située sensiblement au niveau de l'extrémité de la tablette de chargement 4. La paroi 16 présente deux bras 18 qui permettent sa fixation au boîtier 1, et une ouverture 20 qui laisse voir un chargeur 22.

Le chargeur 22 est attaché à la paroi 16 sensiblement au niveau de la tablette de chargement 4, du côté opposé celle-ci. Lorsqu'un portoir 8 est poussé selon une flèche 21 depuis la tablette 4, il est reçu directement dans le chargeur 22. Le chargeur 22 comprend un vérin 24, propre à se déplacer selon la direction Y, qui commande un doigt (non représenté) à travers l'ouverture 20 qui vient pousser un portoir 8 reçu dans le chargeur 22 selon une flèche 25 vers l'intérieur du dispositif 1, comme on le verra plus bas.

Le dispositif 1 comporte également un agitateur 30, un élévateur 32 et un passeur 34. L'agitateur 30 a pour fonction de mélanger les portoirs de tubes 8 qu'il reçoit, par rotation selon un axe sensiblement parallèle à Y.

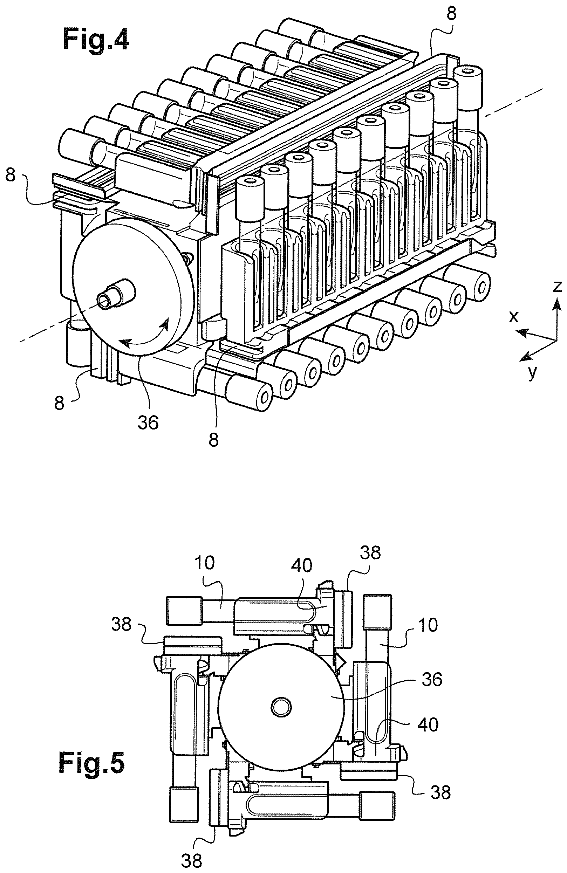

Comme on peut le voir sur les figures 4 et 5, l'agitateur 30 peut recevoir 4 portoirs 8, agencés chacun à environ 90° les uns des autres. En variante, l'agitateur 30 pourrait être agencé pour ne recevoir que 3 portoirs 8, ou plus de 4 portoirs. L'agitateur 30 comprend un moteur 36 qui est agencé pour appliquer une rotation permettant de mélanger le contenu des tubes 10 reçus sur les portoirs 8 qu'il supporte.

Comme on le verra dans la suite, le moteur 36 peut également être commandé pour présenter un portoir 8 choisi en face du passeur 34. Pour recevoir les portoirs 8, l'agitateur 30 comprend quatre supports 38 présentant chacun un guide 40 allongé selon la direction Y. Les supports 38 sont reliés au moteur 36, de sorte que la rotation de ce dernier entraîne la rotation des supports 38 et des portoirs 8 qu'ils reçoivent.

Comme on peut le voir sur les figures 6 et 7, les portoirs 8 présentent une base 42 en forme de T qui vient se loger dans un des guides 40 de l'agitateur 30. Ainsi, pour introduire un portoir 8, il suffit de placer le support 38 voulu de l'agitateur 30 en face du chargeur 22, et le vérin 24 est actionné pour pousser le portoir 8 en place dans le guide 40 correspondant.

L'agitateur 30 est déplacé au sein du dispositif 1 par l'élévateur 32 selon une flèche 35 sur la figure 2. L'élévateur 32 fait évoluer l'agitateur 30 selon l'axe Z entre une position basse dans laquelle il peut recevoir un portoir 8 du chargeur 22 et une position haute dans laquelle le passeur 34 peut prélever un tube 10 sur un portoir 8. L'élévateur 32 est entraîné par une courroie 43 et un moteur 44.

La figure 8 montre une vue en perspective du passeur 34, selon un axe en miroir par rapport à la vue de la figure 2.

Le passeur 34 comprend un moteur 45 qui entraîne une courroie 46 qui est relié à une portion d'attache 47. Le passeur 34 présente des roues 49 qui sont reçues dans un rail 48 visible sur la figure 2 monté sur la paroi 16. Ainsi, le passeur 34 se déplace selon l'axe Y comme représenté par la flèche 50.

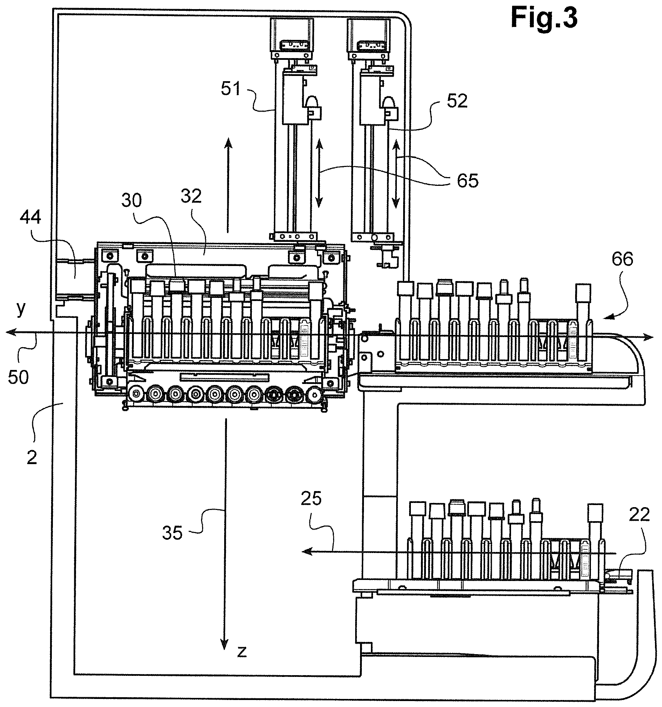

Le passeur 34 a pour fonction principale le prélèvement d'un tube 10 depuis un portoir 8 reçu sur l'agitateur 30, et l'amenée de ce tube 10 vers un premier organe d'échantillonnage 51 ou un deuxième organe d'échantillonnage 52 (visibles sur la figure 3).

Afin de réaliser le prélèvement des tubes 10, le passeur 34 comprend une pince 54 qui est déplacée selon l'axe X au moyen d'un actionneur 56. Pour identifier le tube 10 à prélever, le passeur 34 utilise un capteur 58 qui lit un code barre ou un autre moyen d'identification optique porté par les tubes 10. La pince 54 pourrait également être remplacée par un autre organe de préhension, par exemple un suceur à vide, un organe magnétique, etc. Le capteur 58 pourrait également être différent, et être par exemple un capteur RFID.

Le passeur 34 comprend enfin une butée haute 60 et une butée basse 64 qui limitent le déplacement du tube lors de l'introduction et l'extraction de l'aiguille pour le prélèvement, ainsi qu'un poussoir 62. Le poussoir 62 permet de réaliser l'éjection d'un portoir 8 de l'agitateur 30 vers la tablette de déchargement 6.

Pour emmener un tube 10 de l'agitateur 30 vers un des organes d'échantillonnage 51 et 52, l'élévateur 32 déplace l'agitateur 30 en position haute au niveau du passeur 34, et le moteur 36 de l'agitateur 30 présente le portoir 8 recherché en face du passeur 34. Ensuite, le passeur 34 se déplace selon l'axe Y jusqu'à identifier le tube 10 recherché au moyen du capteur 58. Une fois le tube 10 identifié, l'actionneur 56 déplace la pince 54 qui se saisit du tube 10, et l'élévateur 32 commande une descente de l'agitateur 32 selon la direction Z, de sorte que le tube 10 est extrait du portoir 8. Le passeur 34 est alors déplacé selon la direction Y en dessous du premier organe d'échantillonnage 51 ou du deuxième organe d'échantillonnage 52. Une fois le passeur 34 arrêté, l'aiguille de l'organe d'échantillonnage se déplace selon l'axe Z comme représenté par les flèches 65 pour réaliser le prélèvement dans le tube 10. Enfin par une suite d'opérations inverses, le tube 10 est replacé dans le portoir 8 sur l'agitateur 30.

Pour extraire un portoir 8 de l'agitateur 30, le passeur 34 est déplacé le plus à gauche selon l'axe Y selon la vue des figures 2 et 3. Ensuite, l'élévateur 32 déplace l'agitateur 30 en position haute au niveau du passeur 34, et le moteur 36 de l'agitateur 30 présente le portoir 8 recherché en face du passeur 34. Enfin, le passeur 34 est déplacé vers la droite selon l'axe Y, le poussoir 62 poussant progressivement le portoir 8 en dehors du support 38 qui le reçoit, jusqu'à une zone de déchargement 66 de la tablette de déchargement 6, et un actionneur non représenté déplace le portoir 8 extrait selon l'axe X comme représenté par la flèche 67 pour extraire le portoir 8 du dispositif 1.

Toutes ces actions sont coordonnées par un ordonnanceur qui peut être réalisé sous la forme d'un programme informatique. L'ordonnanceur a pour fonction la coordination du chargeur 22, de l'agitateur 30, de l'élévateur 32 et du passeur 34 ainsi que des organes d'échantillonnage 51 et 52 afin d'introduire les portoirs 8, de les agiter, et de réaliser les prélèvements.

Dans l'exemple décrit ici l'ordonnanceur est intégré au sein du boîtier du dispositif 1. En variante, l'ordonnanceur pourrait être déporté, et être exécuté depuis un ordinateur ou un contrôleur situé hors du boîtier du dispositif 1, et en communication avec ce dernier de manière filaire ou sans fil. De plus, un tel ordinateur ou contrôleur pourrait commander une pluralité de dispositifs 1 auxquels il serait relié, indépendamment les uns des autres.

Lorsqu'un portoir 8 est chargé dans l'agitateur 30, il est déplacé par l'élévateur 32 au niveau du passeur 34, qui utilise le capteur 58 pour déterminer les tubes 10 qui sont présents sur le portoir 8, ainsi que les analyses qui sont requises pour ces tubes.

Ainsi, l'ordonnanceur sait exactement quels tubes sont présents sur les portoirs 8, et peut définir une séquence de prélèvement et d'analyse permettant d'optimiser la cadence du dispositif 1.

En effet, si un tube urgent est introduit par la trappe 14, l'ordonnanceur peut « mettre en pause » la séquence en cours, et préparer le prélèvement du tube urgent dès que le prélèvement du tube en cours est terminé et que celui-ci a été replacé dans son portoir.

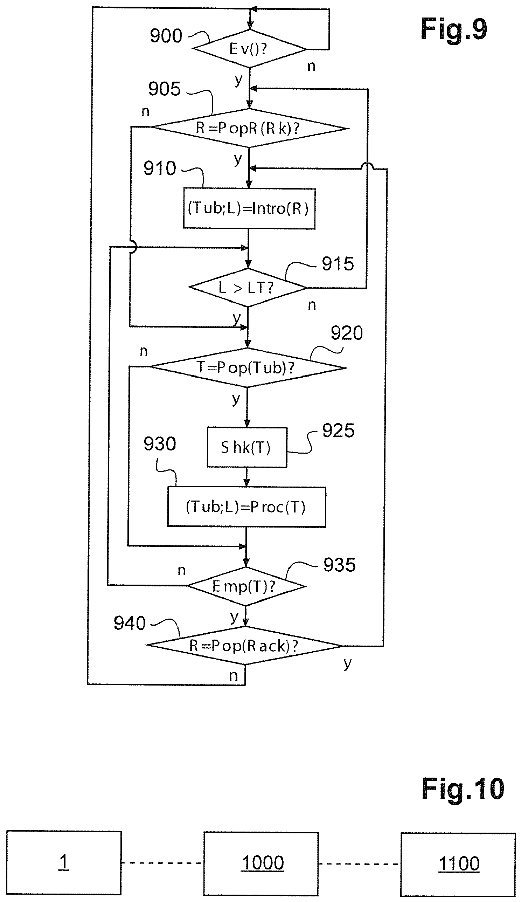

La figure 9 représente un exemple fonction mise en œuvre par l'ordonnanceur.

Cette figure représente une boucle de fonctionnement de l'ordonnanceur, qui commence avec une opération 900 dans laquelle une fonction Ev() teste la présence d'un évènement de lancement d'analyse.

Lorsqu'un tel évènement est détecté, une fonction PopR() est exécutée dans une opération 905. La fonction PopR() reçoit comme argument une liste Rk. La liste Rk contient les identifiants des portoirs 8 de tubes 10 actuellement reçus par le dispositif 1. Dans l'exemple décrit ici, les portoirs 8 sont agencés par ordre de priorité dans la liste Rk, de sorte que le premier portoir 8 est celui qui doit être traité en priorité etc.

La gestion de la liste Rk et de l'introduction des portoirs 8 dans celle-ci peut être gérée par l'ordonnanceur ou être automatisée. Dans l'exemple décrit ici, les portoirs 8 reçus sur la tablette de chargement 4 sont classés par ordre de proximité à l'entrée du dispositif 1, tandis que lorsqu'un portoir urgent ou un tube urgent est introduit, il est placé en tête de la liste Rk. D'autres méthodes de gestion de la liste Rk pourront être utilisées.

La fonction PopR() dépile la liste Rk et l'identifiant de portoir 8 résultant est stocké dans une variable R. Ensuite, dans une opération 910, une fonction Intro() est exécutée. La fonction Intro() reçoit la variable R comme argument et retourne une liste Tub et une variable L.

Dans l'exemple décrit ici, la fonction Intro() commande le dispositif 1 pour introduire le portoir 8 dont l'identifiant est donné par la variable R, ainsi que pour procéder au mélange des tubes 10 qu'il porte.

Dans l'exemple décrit ici, cette introduction comprend la lecture d'étiquettes portées par les tubes 10 permettant de déterminer les identifiants des tubes 10 concernés, ainsi que les analyses qui sont requises. Les identifiants des tubes 10 sont stockés dans la liste Tub pour leur analyse ultérieure. La lecture des tubes 10 permet également de déterminer la durée nécessaire aux analyses à réaliser pour le portoir 8 désigné par la variable R, et cette durée est ajoutée à la variable L, qui reçoit la durée estimée pour analyser tous les tubes chargés dans le dispositif 1.

La fonction Intro() est susceptible de modifier la liste Tub en fonction de l'évolution des traitements depuis l'exécution précédente de la fonction Intro(), en fonction d'un ou plusieurs des critères suivants :
- le type de tests à réaliser sur les tubes nouvellement introduits,
- le statut d'urgence de l'un ou de plusieurs des tubes nouvellement introduits,
- le degré d'agitation des tubes dans le dispositif,
- l'origine du portoir des tubes nouvellement introduits (tablette, tube urgent, etc.),
- le temps de présence de chaque tube présent dans le dispositif.

D'autres critères pourront être pris en compte selon circonstances.

Ensuite, dans une opération 915, un test compare la variable L à un seuil LT. Le seuil LT correspond à la durée estimée pour agiter les tubes d'un portoir 8. En effet, si la durée totale des analyses restantes est inférieure à cette durée (de l'ordre de deux minutes dans l'exemple ici décrit), il est souhaitable de charger un nouveau portoir, afin d'agiter celui-ci le plus vite possible et d'assurer le fonctionnement à cadence maximale du dispositif 1.

Si L est inférieur à LT, alors l'opération 910 est répétée. Dans le cas contraire, l'analyse d'un tube est testée. Pour cela, dans une opération 920, un test détermine si la liste Tub est vide au moyen d'une fonction PopT() qui reçoit la liste Tub comme argument.

Si la liste Tub n'est pas vide, alors la fonction PopT() détermine celui des tubes qui doit être le prochain à être testé. Par exemple, dans le cas d'un portoir urgent ou d'un tube urgent introduit dans l'opération 910, la liste Tub peut recevoir les identifiants de ces tubes dans un emplacement tel que ceux-ci seront traités en premier dès que leur agitation sera terminée. Dans ce cas de figure, la liste Tub est tout simplement dépilée, et l'identifiant du tube correspondant est retourné dans une variable T.

Dans d'autres modes de réalisation, la fonction PopT() peut être plus complexe, et décider en fonction d'algorithmes intrinsèques et/ou extrinsèques le prochain tube à analyser.

Ensuite, dans une opération 925, une fonction Shk() détermine si le tube désigné par la variable T a été suffisamment agité. Si ce n'est pas le cas, alors une agitation supplémentaire est réalisée.

Le tube désigné par la variable T est ensuite analysé dans une opération 930 par une fonction Proc(). La fonction Proc() commande le dispositif 1 pour venir chercher le tube d'identifiant T sur son portoir, pour l'amener jusqu'à l'un des deux organes d'échantillonnage 51 et 52, et pour le replacer dans son portoir une fois l'analyse réalisée. Pour aller chercher le tube d'identifiant T, le dispositif 1 peut commander la lecture des étiquettes des tubes sur le portoir considéré, ou utiliser une table intermédiaire associant chaque tube reçu dans le dispositif 1 à un emplacement donné sur une face de l'agitateur 30. Dans l'exemple décrit ici, l'organe d'échantillonnage 51 est dédié aux analyses directes, tandis que l'organe d'échantillonnage 52 est dédiée aux répétitions de tests.

La fonction Proc() met également à jour la liste Tub, soit en supprimant l'identifiant T, soit en replaçant celui-ci si une répétition de test est nécessaire. La variable L est également mise à jour en conséquence, en soustrayant à celle-ci la durée de l'analyse qui était prévue pour le tube d'identifiant T.

Enfin dans une opération 935, une fonction Emp() détermine si la liste Tub est vide. Si ce n'est pas le cas, alors la boucle reprend avec l'opération 915 pour déterminer si un nouveau portoir doit être chargé. Sinon, dans une opération 940 une opération identique à l'opération 910 est réalisée. S'il n'y a plus de portoir, alors toutes les analyses ont été réalisées, et le dispositif 1 reprend avec l'attente d'un prochain évènement dans l'opération 900. Sinon, la boucle reprend avec l'opération 910 pour introduire ce portoir.

Lorsque l'agitateur 30 présente quatre supports de réception de portoir, l'un d'entre eux peut être laissé vide en permanence afin de servir de tampon qui est utilisé pour stocker un tube de manière temporaire selon les besoins.

Le fait que l'agitateur 30 peut recevoir au moins trois portoirs 8 permet de garantir une cadence optimale en cas de nécessité de répétition de test (également appelé Rerun/Reflex). Ainsi, même si les trois portoirs ne contiennent qu'un tube « utile », la continuité de la cadence est assurée.

En effet, avant de faire l'objet d'un prélèvement, les tubes doivent faire l'objet d'une agitation suffisante afin d'assurer l'homogénéité du mélange dans chaque tube. Cela signifie que, lorsqu'un portoir est introduit dans le dispositif, il faut d'abord agiter les tubes. Or cette opération nécessite l'équivalent de 4 opérations de prélèvements. Par conséquent, lorsque deux portoirs peuvent être reçus, une certaine continuité de cadence peut être assurée en commençant à agiter les tubes du deuxième portoir tandis que les derniers tubes du premier portoir font l'objet d'un prélèvement.

Néanmoins, dans le cas d'un tube urgent ou d'une opération de type Rerun/Reflex, la cadence est susceptible de chuter, le temps d'agiter convenablement ces tubes. Le troisième portoir permet de continuer de traiter un ou deux portoirs, tout en agitant le tube urgent ou le tube d'opération de type Rerun/Reflex. Ainsi, ces derniers sont traités aussi vite que possible, tout en maintenant une cadence de fonctionnement maximale pour le dispositif.

Lorsque l'agitateur 30 reçoit 4 supports ou plus, de nouvelles possibilités s'ouvrent, comme l'utilisation d'un portoir tampon décrite ci-dessus, ou la possibilité de laisser un support vide en permanence, afin de permettre l'utilisation de « portoir urgent », c'est-à-dire non plus un tube, mais un portoir entier de tubes qui seront prélevés et analysés en priorité.

Enfin, le dispositif 1 peut également recevoir un deuxième agitateur similaire à l'agitateur 30. Cela permet l'utilisation de tampons supplémentaires, et l'élévateur 32 et le passeur 34 permettront le transfert d'un portoir à un autre, sur le même agitateur ou entre les deux agitateurs.

Grâce au nouveau paradigme proposé par l'invention, il devient possible de tenir une cadence réelle de 120 prélèvements par heure, même en utilisant des tubes urgents ou en ayant des portoirs à un seul tube utile. Au-delà de la cadence réelle améliorée, la conception du dispositif selon l'invention ouvre un champ nouveau de traitement des analyses, puisqu'il devient possible de déterminer le chemin de prélèvement le plus rapide, indépendamment de la composition des portoirs et de leur ordre d'introduction dans le dispositif 1. En fonction des dimensionnements choisis pour les composants du dispositif, la cadence pourrait être augmentée, par exemple à 240 prélèvements par heure ou plus.

Dans le cadre de l'invention, la Demanderesse a poussé l'analyse mécanique et fonctionnelle des éléments du dispositif afin de lisser le fonctionnement le plus possible.

Cela a amené la Demanderesse à définir des cycles d'action de 15 secondes, et à définir les actions suivantes :
a. - chargement d'un portoir depuis la tablette avant et identification de ce portoir et des tubes qu'il reçoit,
b. - agitation,
c. - vérification d'identifiant d'un tube, préhension de ce tube, fourniture à l'échantillonnage et remise en place de ce tube,
d. - éjection d'un portoir vers la tablette avant.

Les actions a. à d. permettent la gestion simple des interactions au sein d'un dispositif non relié à un convoyeur et non apte à trier les tubes et/ou portoirs. L'ordonnanceur est agencé pour gérer ces actions selon un ordre permettant d'optimiser la cadence de fonctionnement du dispositif.

La Demanderesse a également découvert que ces actions pouvaient être étendues pour automatiser la connexion du dispositif à un convoyeur (référencé 1000 sur la figure 10) permettant de les envoyer vers un autre dispositif d'agitation et de prélèvement (référencé 1100 sur la figure 10) pouvant être conforme au dispositif 1 selon l'invention ou différer de celui-ci, ainsi que pour automatiser le tri des tubes 10 et/ou des portoirs 8 de tubes 10, pour un traitement ultérieur automatisé ou manuel.

Dans ce cas, de nouvelles actions peuvent être définies pour la gestion par l'ordonnanceur :
e. chargement d'un portoir depuis le convoyeur et identification de ce portoir,
f. vérification d'identifiant d'un tube, préhension de ce tube, fourniture à l'échantillonnage puis remise en place de ce tube, et chargement simultané d'un portoir depuis la zone d'attente du convoyeur,
g. vérification d'identifiant d'un tube, préhension de ce tube, fourniture à l'échantillonnage puis remise en place de ce tube, et éjection simultanée d'un portoir vers la zone d'attente du convoyeur,
h. agitation et identification d'un portoir,
i. éjection d'un portoir vers le convoyeur,
j. éjection d'un portoir avec décalage sur la tablette avant,
k. décalage vertical d'un ou plusieurs tubes d'un portoir et éjection de ce portoir sur la tablette avant avec ou sans décalage du portoir,
l. vérification d'identifiant d'un tube, préhension de ce tube, fourniture à l'échantillonnage et remise en place de ce tube à un autre emplacement que celui d'origine (portoir et/ou position sur le portoir),
m. vérification d'identifiant d'un tube, préhension de ce tube, fourniture à l'échantillonnage puis remise en place de ce tube à un autre emplacement que celui d'origine (portoir et/ou position sur le portoir), et chargement simultané d'un portoir (depuis la zone d'attente du convoyeur ou depuis un autre emplacement),
n. vérification d'identifiant d'un tube, préhension de ce tube, fourniture à l'échantillonnage puis remise en place de ce tube à un autre emplacement que celui d'origine (portoir et position sur le portoir), et éjection simultanée d'un portoir (vers la zone d'attente du convoyeur ou vers un autre emplacement), et
o. vérification d'identifiant d'un tube, préhension de ce tube, et remise en place de ce tube à un autre emplacement que celui d'origine (portoir et position sur le portoir).

Les actions e. à i. permettent de gérer la connexion du dispositif de l'invention à une installation plus complexe dans un laboratoire d'analyses. Dans ce cas, le dispositif est susceptible de recevoir des tubes 10 et/ou des portoirs 8 d'autres dispositifs 1100 au sein de l'installation. Cette transmission est réalisée par un convoyeur 1000 qui amène les tubes 10 et/ou portoirs 8 à une zone d'attente dédiée du dispositif 1.

Les actions j. et k. permettent de gérer les tubes et/ou portoirs pour un tri manuel en sortie. En effet, un opérateur voyant en sortie un tube décalé verticalement et/ou un portoir décalé saura que ces derniers doivent faire l'objet d'un traitement distinct, que cela soit une analyse complémentaire ou une ré-analyse. Bien sûr les dimensions du dispositif (longueur de la tablette, hauteur de sortie du portoir) doivent être adaptées en conséquence.

Les actions a. à k. ne nécessitent aucune adaptation de la logique du dispositif décrit plus haut. L'introduction d'un portoir (fonction Intro() de la figure 9), le traitement d'un tube (fonction Proc() de la figure 9) et/ou d'un portoir (fonction Empt() de la figure 9) sont adaptées pour introduire une ou plusieurs des actions a. à k. selon les besoins.

Les actions l. à o. permettent pour leur part de réaliser un triage totalement automatisé des tubes et/ou des portoirs. En effet, en permettant de replacer un tube dans un emplacement distinct de son emplacement d'origine, il devient possible de réorganiser à la volée les tubes sur les différents portoirs pour tenir compte des tests restant à réaliser.

Cela présente plusieurs avantages. Les dispositifs de triage, qu'ils soient dédiés à l'archivage ou à la circulation des tubes entre plusieurs dispositifs d'une même installation sont extrêmement coûteux, occupent beaucoup de place, et nécessitent d'être dimensionnés avec précision par rapport à l'installation.

De plus, si l'installation change de taille (ajout d'autres dispositifs, ou réduction de taille), le dispositif de triage devient inadapté, voire doit être remplacé. Avec l'invention, le dispositif dédié au triage est tout simplement supprimé, car le triage est internalisé. Cela signifie moins de coûts, moins de place, moins de temps de conception (dimensionnement), et une plus grande souplesse d'échelle (quelle que soit la taille de l'installation, le tri est intégré).

Enfin, comme le tri est intégré, un gain de temps considérable peut être réalisé au niveau du remplissage des portoirs. En effet, il n'est plus nécessaire de préparer des portoirs regroupant des tubes avec des analyses similaires à réaliser pour pré-trier les tubes. Les utilisateurs peuvent mettre les tubes en vrac dans les portoirs quelles que soient les analyses, et le dispositif optimisera la répartition des tubes dans les portoirs.

Ce type de traitement automatisé est rendu possible par deux caractéristiques.

La première caractéristique est la capacité de l'agitateur à agiter au moins trois portoirs simultanément, qui permet d'utiliser en permanence un des trois portoirs comme destination d'ajustement.

Ainsi, dès qu'il est déterminé qu'un tube doit faire l'objet d'une analyse différente des tubes de son portoir d'origine après l'analyse courante, celui-ci est replacé après analyse sur le portoir d'ajustement. Dès qu'un autre tube est déterminé comme devant faire l'objet de la même analyse, il est également replacé sur ce portoir, et ainsi de suite jusqu'à ce que le portoir d'ajustement soit rempli ou qu'un seuil de temps soit dépassé, suite à quoi le portoir d'ajustement est extrait du dispositif, vers le convoyeur s'il y en a un, ou avec décalage sur la tablette pour indiquer à l'utilisateur la destination de ce portoir.

Lors du fonctionnement classique du dispositif, le nombre total d'emplacements « vides » sur les portoirs reçus (c'est-à-dire le nombre d'emplacements ne recevant pas de tube), est constant, puisque les tubes qui sont déplacés sur un autre portoir ou à un autre emplacement que leur emplacement d'origine laissent ce dernier vide. Cela reste le cas tant les portoirs d'ajustement sont éjectés dès qu'ils sont pleins et qu'ils sont remplacés par un autre portoir plein en entrée.

Lorsque le portoir d'ajustement est extrait sur requête de l'utilisateur ou suite à dépassement d'un seuil de temps, un portoir vide doit alors être introduit dans le dispositif pour permettre de recouvrer la capacité d'ajustement.

D'une manière générale, chaque portoir supplémentaire géré par l'agitateur permet de géré une destination d'ajustement différente. Quatre portoirs permettront donc de gérer deux destinations d'ajustement, et l'ajout d'un second agitateur permettra d'augmenter le nombre de destinations d'ajustement.

La seconde caractéristique est un changement dans la gestion de la logique du dispositif. En effet, dans le cas décrit en référence aux actions a. à k., le dispositif est totalement indépendant du reste de la chaîne, puisqu'un portoir ressortira comme il est rentré, même si les tubes qu'il contient ne seront pas forcément traités dans leur ordre dans le portoir ou si un autre portoir pourra être traité en priorité. Dit autrement, le dispositif constitue un univers fermé non influencé par les autres dispositifs, même lorsqu'il est connecté à un convoyeur.

Dans le cas des actions l. à o., le dispositif est un maillon d'une chaîne formée par l'installation. À ce titre, la gestion par le dispositif est influencée par l'état général de l'installation.

Ainsi, lorsqu'un portoir est introduit dans le dispositif, ce dernier lit les informations relatives à chacun des tubes de ce portoir, en tire pour chacun une donnée de destination, et met à jour la liste d'actions à exécuter pour chaque tube. La donnée de destination indique le prochain dispositif vers lequel un tube doit être transporté une fois traité, ou l'archivage s'il n'y a pas d'analyse supplémentaire. Dans l'exemple décrit ici, la donnée de destination est déterminée à partir de l'identifiant du tube en accédant à une base de données locale ou déportée. En variante, elle peut faire partie des informations lues sur le tube.

Ensuite, lorsque vient le moment de réaliser l'analyse d'un tube donné, c'est-à-dire au moment où le dispositif commande à la pince d'aller saisir le tube sur le portoir, une nouvelle lecture des informations relatives au tube est faite pour déterminer si la donnée destination de ce dernier après l'analyse courante est identique à celle du portoir qui le reçoit. Si c'est le cas, alors c'est l'action c., f., ou g. qui est exécutée selon ce qui est prévu pour optimiser la cadence. Sinon, c'est l'action l., m. ou n qui est exécutée.

Initialement, aucun portoir n'a de donnée de destination. C'est lors du prélèvement du premier tube qu'il reçoit qu'il peut se voir affecter une donnée de destination. En effet, selon l'invention, lorsqu'aucun portoir ne présente de donnée de destination identique à celle d'un tube, le portoir le plus ancien se voit donner cette donnée de destination.

En variante, les portoirs pourraient se voir affecter une donnée de destination en fonction des données de destination des tubes qu'ils reçoivent ainsi que des données de destination des autres portoirs déjà chargés lors du chargement du portoir dans le dispositif.

Ainsi, au fur et à mesure que les tubes sont traités, la disposition des tubes et leur répartition sur les portoirs chargés dans le dispositif sont modifiées. Par exemple, lorsqu'un tube a été replacé dans un portoir différent de son portoir d'origine, le tube suivant du portoir d'origine, s'il est replacé dans le portoir d'origine, ne sera pas remis à son emplacement d'origine mais à l'emplacement laissé vide par le tube précédent.

De manière semblable, lorsqu'un tube d'un portoir donné ne doit pas faire l'objet d'une analyse mais que sa donnée de destination est différente de celle du portoir qui le reçoit, un déplacement spécifique du tube par la pince peut être effectué (action o.). C'est également le cas si un tube reçu sur un portoir déjà chargé dans le dispositif voit son analyse être modifiée et ne plus correspondre à la ou les analyses pour lesquelles le dispositif est prévu.

Parmi les actions décrites ci-dessus, l'action o. est la seule susceptible d'impacter la cadence dans la mesure où elle ne prévoit aucune analyse. Cependant, ce type de situation devrait être très rare dans la pratique, et ne peut pas être évité.

Il apparaît donc que, si le dispositif fonctionne selon les mêmes principes que ceux exposés précédemment, et permet d'obtenir des avantages similaires, sa logique est différente car des événements extérieurs sont susceptibles d'impacter son fonctionnement.

Par exemple, l'utilisateur peut décider d'ajouter un test complémentaire non prévu à l'origine pour un tube déjà dans l'installation. Dans ce cas, ce tube sera très vraisemblablement déplacé vers un portoir de destination d'ajustement au cours de l'un de ses traitements. De plus, tous les tubes suivants seront également déplacés vers un emplacement distinct de leur emplacement originel. Malgré cela, grâce au dispositif de l'invention, la cadence restera maximale.

Les exemples donnés pour la mise en œuvre des opérations l. à o. ont été décrits en référence à une installation comprenant plusieurs dispositifs reliés par un convoyeur. Cependant, elles sont entièrement combinables avec les opérations j. et k. en l'absence d'un convoyeur. En effet, les portoirs ayant des données de destination choisies pourront être décalés sur la tablette 4 ou 6 selon une distance caractéristique de la destination concernée. En outre, l'information de destination correspondante pourra être affichée à proximité pour chaque portoir.

Dans ce qui précède, la durée des opérations a été fixée à 15 secondes, ce qui offre un bon compromis entre granularité des opérations et efficacité d'ordonnancement, notamment en vue de la cadence recherchée de 120 prélèvements par heure. En variante, d'autres durées d'actions pourront être retenues, en fonction des compromis et des cadences recherchés.

D'une manière générale, à chaque fois qu'un portoir qui avait été introduit vide dans le dispositif est sorti « de force » du dispositif, c'est-à-dire sur requête de l'utilisateur, ou suite au dépassement d'un seuil de temps, que ce soit vers un convoyeur ou vers une tablette, il doit être remplacé. Ce remplacement peut être manuel ou automatisé (par exemple via le convoyeur).

Le dispositif qui a été décrit ci-dessus est prévu pour réaliser un certain nombre d'analyses après l'échantillonnage des tubes. En variante, le dispositif pourrait être limité au seul échantillonnage, et l'analyseur être déporté.

La description qui précède a été réalisée en référence à des produits sanguins, cependant l'invention trouve son application avec le traitement de tous types de liquides biologiques et en particulier les fluides corporels.

## Revendications

1. Dispositif d'agitation et de prélèvement d'échantillons de liquides biologiques comprenant un agitateur (30) de portoirs (8) de tubes (10), un organe d'échantillonnage (51, 52) propre à réaliser un prélèvement d'échantillon de liquides biologiques dans un tube (10), et un passeur (34) capable de saisir un tube (10) sur un portoir (8) reçu dans l'agitateur (30) et de le déplacer jusqu'à l'organe d'échantillonnage (51, 52), **caractérisé en ce que** l'agitateur (30) est capable d'agiter au moins trois portoirs (8) simultanément, et **en ce qu'**il comporte en outre un ordonnanceur capable de déterminer des données de destination pour un tube (10) et des données de destination pour le portoir (8) qui reçoit ce tube (10), et de déterminer pour chaque tube (10) un emplacement final en fonction des données de destination du tube (10) et des données de destination des portoirs (8) reçus dans le dispositif, lequel emplacement final désigne un portoir (8) reçu sur l'agitateur (30) et une position sur ce portoir (8) et peut être différent de l'emplacement du tube (10) lorsque le portoir (8) qui le recevait a été introduit dans le dispositif (1), et agencé pour commander le passeur (34) pour saisir un tube (10), le présenter à l'organe d'échantillonnage (51, 52) et le replacer après prélèvement audit emplacement final.

2. Dispositif selon la revendication 1, dans lequel l'ordonnanceur est agencé, pour un tube (10) donné, pour choisir l'emplacement final parmi les emplacements libres sur un portoir (8) qui présente les mêmes données de destination que le tube (10) donné.

3. Dispositif selon la revendication 2, dans lequel l'ordonnanceur est agencé pour déterminer comme emplacement final sur un portoir (8) donné le premier emplacement vide sur le portoir (8) donné.

4. Dispositif selon l'une des revendications précédentes, dans lequel l'ordonnanceur est agencé pour mettre à zéro les données de destination associées à un portoir (8) donné lors de son introduction dans le dispositif (1).

5. Dispositif selon l'une des revendications précédentes, dans lequel l'ordonnanceur est agencé, lorsqu'aucun portoir (8) ne présente les mêmes données de destination qu'un tube (10) saisi par le passeur (34), pour affecter les données de destination associées à ce tube (8) à un portoir (8) auquel aucune donnée de destination n'est associée.

6. Dispositif selon l'une des revendications précédentes, dans lequel l'ordonnanceur est agencé pour commander le passeur (34) pour décaler verticalement un tube (10) reçu dans un portoir (8) en fonction des données de destination associées à ce tube (10).

7. Dispositif selon l'une des revendications précédentes, dans lequel l'ordonnanceur est agencé pour commander le passeur (34) pour faire sortir un portoir (8) du dispositif (1), et pour le disposer en une position de sortie variant en fonction des données de destination associées au portoir (8).

8. Dispositif selon l'une des revendications précédentes, dans lequel l'ordonnanceur est agencé pour faire sortir un portoir (8) du dispositif (1) présentant des emplacements vides, en réponse à une commande utilisateur ou en réponse au dépassement d'une durée choisie.

9. Dispositif selon la revendication 7 ou 8, dans lequel l'ordonnanceur est agencé, après avoir fait sortir un portoir (8) présentant des emplacements vides, pour faire rentrer dans le dispositif (1) un portoir (8) présentant un nombre d'emplacements vides au moins égal.

10. Dispositif selon l'une des revendications précédentes, comprenant en outre une zone pour connexion à un convoyeur de portoirs (8).

11. Procédé d'agitation et de prélèvement de tubes, réalisé au moyen du dispositif selon l'une des revendications 1 à 10, comprenant les opérations suivantes :
a. introduire au moins un portoir (8) recevant au moins un tube (10) contenant au moins un liquide biologique dans un dispositif (1) d'agitation et de prélèvement d'échantillons de liquides biologiques,
b. déterminer des données de destination pour chaque tube (10) dans le dispositif (1) et des données de destination pour chaque portoir (8) qui reçoit un de ces tubes (10),
c. déterminer pour chaque tube (10) reçu dans le dispositif (1) un emplacement final en fonction des données de destination du tube (10) et des données de destination des portoirs (8) reçus dans le dispositif (1), lequel emplacement final désigne un portoir (8) reçu dans le dispositif (1) et une position sur ce portoir (8), et peut être différent de l'emplacement du tube (10) lorsque le portoir (8) qui le recevait a été introduit dans le dispositif (1)
d. réaliser le prélèvement d'échantillons de liquides biologiques dans chaque tube (10) reçu dans le dispositif (1),
e. placer le tube (10) à son emplacement final après l'opération d).

## Patentansprüche

1. Vorrichtung zum Schütteln und Entnehmen biologischer Flüssigkeitsproben, umfassend eineinen Schüttler (30) von Trägereinrichtungen (8) von Röhrchen (10), ein Probenahmeorgan (51, 52), das geeignet ist, eine Entnahme einer Probe biologischer Flüssigkeiten in einem Röhrchen (10) durchzuführen, und eine Übergabeeinrichtung (34), die imstande ist, ein Röhrchen (10) an einer Trägereinrichtung (8) zu ergreifen, die in dem Schüttler (30) aufgenommen ist, und es bis zum Probenahmeorgan (51, 52) zu bewegen, **dadurch gekennzeichnet, dass** der Schüttler (30) imstande ist, mindestens drei Trägereinrichtungen (8) gleichzeitig zu schütteln, und dadurch, dass sie weiter einen Anweiser beinhaltet, der imstande ist, Zieldaten für ein Röhrchen (10) und Zieldaten für die Trägereinrichtung (8), die dieses Röhrchen (10) aufnimmt, zu bestimmen, und für jedes Röhrchen (10) eine endgültige Stelle in Abhängigkeit von den Zieldanten des Röhrchens (10) und den Zieldaten der Trägereinrichtungen (8) zu bestimmen, die in der Vorrichtung aufgenommen sind, wobei die endgültige Stelle eine Trägereinrichtung (8), die an dem Schüttler (30) aufgenommen ist, und eine Position in dieser Trägereinrichtung (8) bezeichnet, und anders als die Stelle des Röhrchens (10) sein kann, wenn die Trägereinrichtung (8) die es aufgenommen hat, in die Vorrichtung (1) eingeführt worden ist, und angeordnet, um die Übergabeeinrichtung (34) zu steuern, um ein Röhrchen (10) zu ergreifen, es dem Probenahmeorgan (51, 52) vorzulegen und es nach Entnahme an die endgültige Stelle zurückzustellen.

2. Vorrichtung nach Anspruch 1, wobei der Anweiser angeordnet ist, um für ein gegebenes Röhrchen (10) die endgültige Stelle aus den freien Stellen an einer Trägereinrichtung (8) auszuwählen, die dieselben Zieldaten wie das gegebene Röhrchen (10) aufweist.

3. Vorrichtung nach Anspruch 2, wobei der Anweiser angeordnet ist, um als endgültige Stelle an einer gegebenen Trägereinrichtung (8) die erste leere Stelle in der gegebenen Trägereinrichtung (8) zu bestimmen.

4. Vorrichtung nach einem der vorstehenden Ansprüche, wobei der Anweiser angeordnet ist, um die Zieldaten, die einer gegebenen Trägereinrichtung (8) bei ihrer Einführung in die Vorrichtung (1) zugeordnet werden, auf null zu setzen.

5. Vorrichtung nach einem der vorstehenden Ansprüche, wobei der Anweiser angeordnet ist, um wenn keine Trägereinrichtung (8) dieselben Zieldaten wie ein Röhrchen (10) aufweist, das von der Übergabeeinrichtung (34) ergriffen wird, die Zieldaten, die diesem Röhrchen (8) zugeordnet sind, einer Trägereinrichtung (8) zuzuteilen, der kein Zieldatum zugeordnet ist.

6. Vorrichtung nach einem der vorstehenden Ansprüche, wobei der Anweiser angeordnet ist, um die Übergabeeinrichtung (34) zu steuern, um ein Röhrchen (10), das in einer Trägereinrichtung (8) aufgenommen wird, in Abhängigkeit von den Zieldaten, die diesem Röhrchen (10) zugeordnet sind, vertikal zu verschieben.

7. Vorrichtung nach einem der vorstehenden Ansprüche, wobei der Anweiser angeordnet ist, um die Übergabeeinrichtung (34) zu steuern, um eine Trägereinrichtung (8) aus der Vorrichtung (1) austreten zu lassen, und um sie in einer Austrittsposition anzuordnen, die in Abhängigkeit von den Zieldaten, die der Trägereinrichtung (8) zugeordnet sind, variiert.

8. Vorrichtung nach einem der vorstehenden Ansprüche, wobei der Anweiser angeordnet ist, um eine Trägereinrichtung (8), die leere Stellen aufweist, als Reaktion auf eine Nutzersteuerung oder als Reaktion auf die Überschreitung einer ausgewählten Dauer aus der Vorrichtung (1) austreten zu lassen.

9. Vorrichtung nach Anspruch 7 oder 8, wobei der Anweiser angeordnet ist, um, nachdem er eine Trägereinrichtung (8), die leere Stellen aufweist, hat austreten lassen, eine Trägereinrichtung (8) in die Vorrichtung (1) eintreten zu lassen, die eine mindestens gleiche Anzahl an leeren Stellen aufweist.

10. Vorrichtung nach einem der vorstehenden Ansprüche, weiter eine Zone zum Verbinden mit einem Förderer von Trägereinrichtungen (8) umfassend.

11. Verfahren zum Schütteln und Entnehmen von Röhrchen, das anhand der Vorrichtung nach einem der Ansprüche 1 bis 10 ausgeführt wird, die folgenden Vorgänge umfassend:
a. Einführen mindestens einer Trägereinrichtung (8), die mindestens ein Röhrchen (10) aufnimmt, das mindestens eine biologische Flüssigkeit enthält, in eine Vorrichtung (1) zum Schütteln und Entnehmen biologischer Flüssigkeitsproben,
b. Bestimmen von Zieldaten für jedes Röhrchen (10) in der Vorrichtung (1) und von Zieldaten für jede Trägereinrichtung (8), die eines dieser Röhrchen (10) aufnimmt,
c. Bestimmen für jedes Röhrchen (10), das in der Vorrichtung (1) aufgenommen wird, einer endgültigen Stelle in Abhängigkeit von den Zieldaten des Röhrchens (10) und den Zieldaten der Trägereinrichtungen (8), die in der Vorrichtung (1) aufgenommen werden, wobei die endgültige Stelle eine Trägereinrichtung (8) bezeichnet, die an der Vorrichtung (1) aufgenommen ist, und eine Position in dieser Trägereinrichtung (8), und anders als die Stelle des Röhrchens (10) sein kann, wenn die Trägereinrichtung (8) die es aufgenommen hat, in die Vorrichtung (1) eingeführt worden ist
d. Durchführen der Entnahme biologischer Flüssigkeitsproben in jedem Röhrchen (10), das in der Vorrichtung (1) aufgenommen ist,
e. Platzieren des Röhrchens (10) an seiner endgültigen Stelle nach dem Vorgang d).

## Claims

1. A device for agitating and collecting biological liquid samples comprising an agitator (30) of racks (8) of tubes (10), a sampling means (51, 52) capable of collecting a biological liquid sample in a tube (10), and a changer (34) capable of gripping a tube (10) on a rack (8) received in the agitator (30) and moving it to the sampling means (51, 52), **characterized in that** the agitator (30) is capable of agitating at least three racks (8) simultaneously, and **in that** it further comprises a scheduler capable of determining destination data for a tube (10) and destination data for the rack (8) which receives this tube (10), and of determining for each tube (10) a final location based on the destination data of the tube (10) and the destination data of the racks (8) received in the device, which final location designates a rack (8) received on the agitator (30) and a position on this rack (8) and can be different from the location of the tube (10) when the rack (8) that received it has been introduced into the device (1), and arranged to control the changer (34) in order to grip a tube (10), present it to the sampling means (51, 52) and replace it after sampling at said final location.

2. The device as claimed in claim 1, in which the scheduler is arranged, for a given tube (10), to choose the final location from among the free locations on a rack (8) that has the same destination data as the given tube (10).

3. The device as claimed in claim 2, in which the scheduler is arranged to determine, as a final location on a given rack (8), the first empty location on the given rack (8).

4. The device as claimed in one of the preceding claims, in which the scheduler is arranged to reset the destination data associated with a given rack (8) when the latter is introduced into the device (1).

5. The device as claimed in one of the preceding claims, in which, when no rack (8) presents the same destination data as a tube (10) gripped by the changer (34), the scheduler is arranged to assign the destination data associated with this tube (8) to a rack (8) to which no destination data is associated.

6. The device as claimed in one of the preceding claims, in which the scheduler is arranged to control the changer (34) in order to shift vertically a tube (10) received in a rack (8) according to the destination data associated with this tube (10).

7. The device as claimed in one of the preceding claims, in which the scheduler is arranged to control the changer (34) in order to output a rack (8) from the device (1), and to arrange it in an output position that varies as a function of the destination data associated with the rack (8).

8. The device as claimed in one of the preceding claims, in which the scheduler is arranged to output a rack (8) from the device (1) having empty locations, in response to a user command or in response to the passing of a chosen duration.

9. The device as claimed in claim 7 or 8, in which, after a rack (8) having empty locations has been output, the scheduler is arranged to introduce into the device (1) a rack (8) having an at least equal number of empty locations.

10. The device as claimed in one of the preceding claims, further comprising a zone for connection to a conveyor of racks (8).

11. A process for agitating and sampling tubes performed by means of the device according to any of claims 1 to 10, comprising the following steps:
a. insertion of at least one rack (8), receiving at least one tube (10) containing at least one biological liquid, into a device (1) for agitating and collecting biological liquid samples,
b. determination of destination data for each tube (10) in the device (1) and of destination data for each rack (8) which receives one of these tubes (10),
c. for each tube (10) received in the device (1), determination of a final location based on the destination data of the tube (10) and the destination data of the racks (8) received in the device (1), which final location designates a rack (8) received in the device (1) and a position on this rack (8), and can be different from the location of the tube (10) when the rack (8) that received it has been introduced into the device (1),
d. collection of biological liquid samples in each tube (10) received in the device (1),
e. placement of the tube (10) in its final location after step d).
